# EUROPEAN PATENT SPECIFICATION

(11) **EP 3 620 125 B1**
(45) Date of publication and mention of the grant of the patent: **29.03.2023**
(21) Application number: 19195909.7
(22) Date of filing: 06.09.2019
(51) Int. Cl.: A61B 17/70, A61B 17/80, A61B 17/86

(54) **SYSTEMS BONE FIXATION ANCHOR, PLATE, AND SPACER DEVICES**
SYSTEME FÜR KNOCHENFIXATIONSANKER, PLATTE UND ABSTANDSVORRICHTUNGEN
SYSTÈMES POUR DES DISPOSITIFS D'ANCRAGE, DE PLAQUE ET D'ESPACEMENT DE FIXATION OSSEUSE

(30) Priority: 06.09.2018 US 201816122995
(43) Date of publication of application: 11.03.2020
(73) Proprietor: Globus Medical, Inc., Audubon, PA 19403 (US)
(72) Inventor: FLINT, Brian, Fort Washington, PA 19034 (US); TIONGSON, Christna, Norristown, PA 19401 (US); GAULT, James, Philadelphia, PA 19130 (US)
(74) Representative: Morabito, Sara

(56) References cited:
- EP-A1- 3 348 218
- US-A1- 2013 096 631
- US-A1- 2014 053 696
- US-A1- 2018 049 787

## Description

### FIELD

The present invention relates to a bone fixation plate and fastener systems used to stabilize vertebrae and other bony anatomy. More specifically, the present invention relates to systems for locking a fastener into a bone plate.

### BACKGROUND

Bones and bony structures are susceptible to a variety of weaknesses that can affect their ability to provide support and structure. Weaknesses in bony structures may have many causes, including degenerative diseases, tumors, fractures, and dislocations. Advances in medicine and engineering have provided doctors with a plurality of devices and techniques for alleviating or curing these weaknesses.

In the field of orthopedic surgery, and more specifically spinal surgery, bone fasteners may be used for fixation or for the fastening of orthopedic devices or instruments to bone tissue. An exemplary use of bone fastener may include using the bone fastener to fasten an orthopedic device, such as a bone plate, a spinal spacer, and/or a combination thereof, to a vertebral body for the treatment of a deformity or defect in a patient's spine. Bone fasteners can be secured to a number of vertebral bodies and a bone plate can then be connected to the vertebral bodies via the bone fasteners to fuse a segment of the spine. In another example, bone fasteners can be used to fix the location of a spinal spacer once the spacer is implanted between adjacent vertebral bodies. In yet another example, bone fasteners can be fastened to a number of vertebral bodies to anchor a spinal rod in place along a spinal column to treat a spinal deformity.

In the case of severely weakened bone, surgeons may face challenges in finding proper purchase of the bone fastener into the bone and proper attachment to the bone plate.

A bone fixation system with the features as defined in the preamble of claim 1 is known from EP 3348218 A1.

Therefore, to overcome disadvantages noted above, the present disclosure provides bone fixation systems and methods using bone fasteners with threaded heads to engage and deform a textured portion of a bone plate.

### SUMMARY

The invention is defined in claim 1 while preferred embodiments are set forth in the dependent claims.

Associated surgical methods are also described herein to aid understanding the invention. These methods do not form part of the claimed invention.

To meet this and other needs, the present disclosure provides a bone fixation system having a bone plate and a locking fastener. The bone plate includes an upper surface and a lower surface to be in contact with a bone. The bone plate also includes a locking hole extending from the upper surface to the lower surface, the locking hole includes a textured portion. The textured portion includes a texture that is a non-threaded surface. The locking fastener includes head portion and a shaft portion and the locking fastener is to be received by the locking hole and to inserted into the bone. The head portion is threaded and contains a textured area that is tapered and with dual lead threads and configured to engage the textured portion of the locking hole, wherein an outside diameter of the locking fastener is 5,5 mm.

The present disclosure also provides a bone fixation system including a spacer (not claimed) that may be inserted in between two adjacent vertebral bodies and a bone plate that may engage the spacer. The bone plate may have an upper surface and a lower surface that may be in contact with bone. The bone plate may further have a locking hole extending from the upper surface to the lower surface, the locking hole may include a textured portion and non-textured portion. The textured portion may include a texture that is a non-threaded surface. The bone fixation system may include a locking fastener that may be received in the locking hole and that may be inserted into the bone. The locking fastener may have a threaded head portion configured to lock to the bone plate. The threaded head portion may deform the textured portion of locking hole.

Further, it is also described but not claimed a bone fixation system having a bone plate and a locking fastener, the bone plate including an upper surface and a lower surface that may be in contact with a bone, the bone plate also includes a locking hole extending from the upper surface to the lower surface, the locking fastener includes a head portion and a shaft portion and it is configured to be received by the locking hole and may be inserted into the bone, head portion of the locking fastener is threaded, wherein the locking hole includes a textured portion having a desired texture with a plurality of recesses and projections the texture has a different shape than the threading of the locking hole, the locking fastener being configured to engage the texture of textured area of the locking hole so that when the locking fastener is inserted into the locking hole it engages with the texture thereof and deforms the texture of the locking hole.

The textured portion has a texture that is a non-threaded surface.

In a non claimed example, the textured portion has a texture that is a thread that has a shape different from the thread of the locking fastener.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings wherein:
FIG. 1 illustrates an exemplary bone plate fixation device to used according to the principles of the present disclosure.
FIGS. 2 and 3 illustrate exemplary locking fasteners consistent with the principles of the present disclosure.
FIGS. 4 and 5 illustrate an exemplary bone plate consistent with the principles of the present disclosure.
FIGS. 6 and 7 illustrate exemplary bone plates and locking fasteners consistent with the principles of the present disclosure.
FIGS. 8-10 illustrates exemplary locking mechanisms consistent with the principles of the present disclosure.

### DETAILED DESCRIPTION

Embodiments of the disclosure are generally directed to systems for bone stabilization. Specifically, embodiments are directed to bone plating with locking and/or non-locking fasteners for engaging with a bone fastener. The hole designs may allow for fixed angle and/or variable angle fixation. Systems disclosed herein may allow for locking bone screws into a spinal plate or integrated plate-spacer to create a rigid construct and prevent back-out of screws while maintaining a preferred screw trajectory using a spherical or conical screw head profile and with tapering dual-lead threads at a specified torque. Some embodiments further include locking fasteners with self-forming threads configured to displace the plate material, thereby locking the fastener to the plate.

The present disclosure relates to exemplary embodiments of locking screws into a plate or integrated plate spacer. This is accomplished through interference/cross-threading of tapered dual-lead threads on a head of the locking screw engaging with a screw hole or socket with a series of helical sweeps, diamond knurls, or similar relief cuts arranged about the central axis of the screw hole or socket. Tightening of the dual-lead threads of the screw head into these relief cuts creates rigid fixation of the screw and prevents loosening and screw back-out. As an example, the material of the screw may be the same as the plate/integrated-plate spacer or a harder material to promote controlled deformation and rigid fixation. The helical sweeps, diamond knurls, or similar relief cuts may allow for both fixed and variable angle locking screws with conical variability up to 10 degrees.

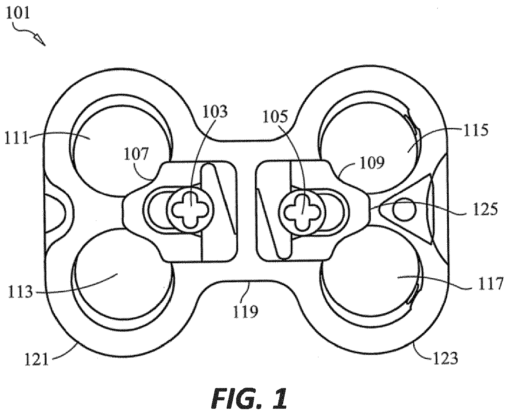

The plate(s) and/or plate-spacer device(s) may be adapted to contact one or more vertebral bodies. The configuration of the locking screw and/or screw hole of the present disclosure may be used by various plates and plate-spacer devices known in the art. Such exemplary bone plates and plate-spacer devices have been described, for example, in U.S. 9,326,802; U.S. 9,095,387; and U.S. 9,044,275. For purposes of illustration, one exemplary bone fixation plate is described in FIG. 1.

FIG. 1 shows one example of a bone fixation plate 101 that may be used with the locking screw/screw hole configuration of the present disclosure. The plate may be secured to two vertebrae in order to maintain the vertebrae integrally with respect to one another in a desired orientation and at a desired spacing from one another. Plate 101 may include two fastening devices, such as screws 103-105 or the like, which are operatively communicable with plates 107-109. The plate also includes four openings 111-117, through which screws (not shown) may be used to fasten the plate 101 to the vertebrae.

Plate 101 and the screws may be comprised of any material, such as a metal, alloy, or any combination of the two. Preferably, the material used to construct the plate and the screws allows the plate 101 to maintain its structural integrity while allowing for a desired amount of resiliency. Furthermore, the material used is preferably bio-compatible and capable of withstanding the conditions of a body over a desired period of time. In some examples, this is achieved by manufacturing the plate 101 and screws using metals such as titanium or stainless steel. Titanium has sufficient ductility to permit a desired amount of curving of the plate 101 to conform to the shape of the vertebrae, yet has the strength to maintain its structural integrity.

In the example of FIG. 1, bone fixation plate 101 comprises a center portion 119 and two distal portions 121-123. Each distal portion 121-123 may be attached to a different vertebra using fasteners, such as screws, that pass through openings 111-117.

Bone plates may be comprised of titanium, stainless steel, cobalt chrome, carbon composite, plastic or polymer-such as polyetheretherketone (PEEK), polyethylene, ultra-high molecular weight polyethylene (UHMWPE), resorbable polylactic acid (PLA), polyglycolic acid (PGA), combinations or alloys of such materials or any other appropriate material that has sufficient strength to be secured to and hold bone, while also having sufficient biocompatibility to be implanted into a body. Similarly, the fasteners may be comprised of titanium, cobalt chrome, cobalt-chrome-molybdenum, stainless steel, tungsten carbide, combinations or alloys of such materials or other appropriate biocompatible materials. Although the above list of materials includes many typical materials out of which bone plates and fasteners are made, it should be understood that bone plates and fasteners comprised of any appropriate material are contemplated.

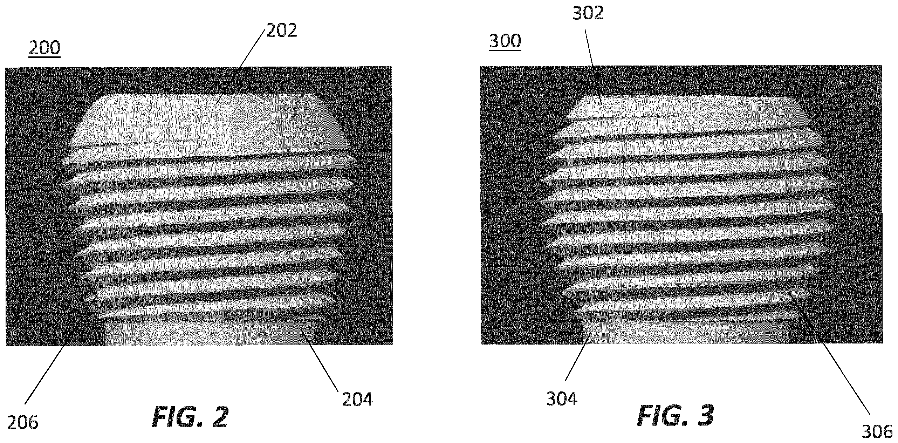

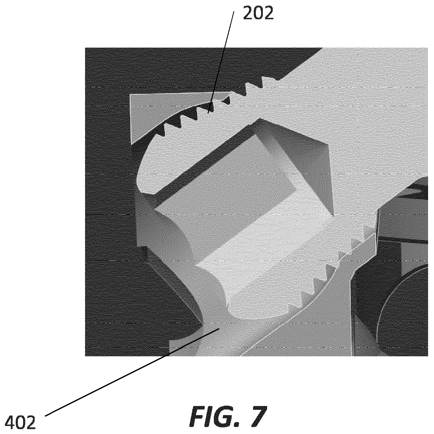

Referring now to FIGS. 2-10, exemplary embodiments of the present disclosure are presented. With regards to FIGS. 2-6, one or more locking fasteners may be inserted into a plate or integrated plate spacer through interference/cross-threading of tapered dual-lead threads on the screw head with a screw hole or socket with a series of helical sweeps, diamond knurls, or similar relief cuts arranged about the central axis of the screw hole or socket. Tightening of the dual-lead threads of the screw head into these relief cuts creates rigid fixation of the screw and prevents loosening and screw back-out.

The material of the screw may be the same as the plate/integrated-plate spacer or a harder material to promote controlled deformation and rigid fixation. The helical sweeps, diamond knurls, or similar relief cuts allow for both fixed and variable angle locking screws with conical variability up to 10 degrees.

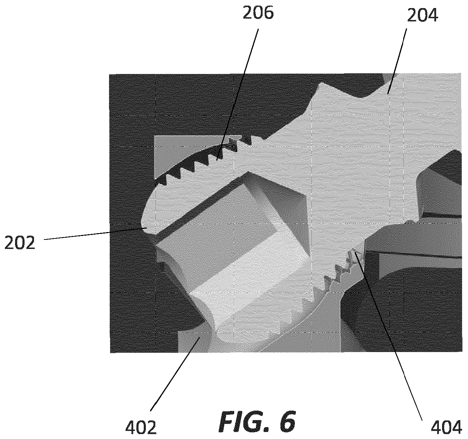

Specifically, locking fastener 200 of FIG. 2 includes a head portion 202 and a shaft portion 204 (shown in FIG. 6) configured to engage bone. Locking fastener 200 has a conical screw head profile with a textured area 206 with dual-lead threads. FIG. 3 illustrates a locking fastener 300 that has a screw head 302 and textured area 306. Screw head 302 has a spherical head profile with dual-lead threads. Each of bone screws 200 and 300 may be used under the principles of this disclosure according to any specific needs of a medical procedure and the preferences of a surgeon performing the medical procedure.

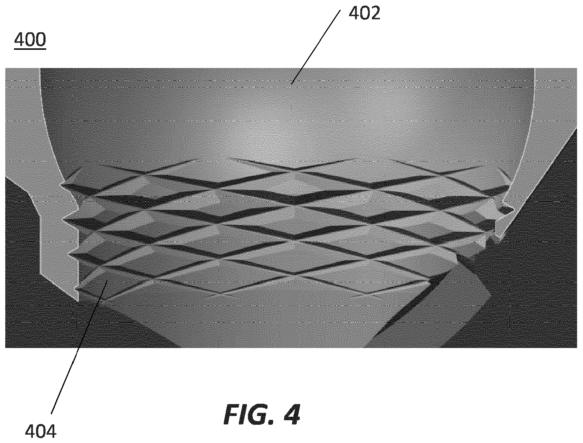

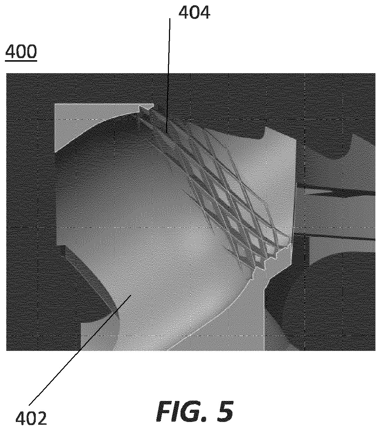

FIGS. 4 and 5 depict a plate 400 with a locking hole 402. An inside surface of screw hole 402 has a textured portion 404. The textured portion 404 comprises a texture that is a non-threaded surface. The textured portion may be, but not limited to, a series of helical sweeps, diamond knurls, threads, ridges, bumps, dimples, serrations or similar relief cuts arranged about the central axis of the screw hole or socket. The textured portion 404 is different from the textured area 206 of locking fastener 200 so that when the locking fastener is inserted into the locking hole 402, the locking fastener deforms the textured portion 404. As shown, the textured portion 404 is disposed along an inner portion of the hole 402. The knurled surface may include straight, angled, or crossed lines cut or rolled into the material. For example, the textured portion 404 may take up about half or less of the surface area of hole 402.

In another version the textured portion 404 is of the same type (e.g., mating surfaces) the textured area 206 of locking fastener 200.

An upper portion of the hole 402 may be tapered, without texturing, for example, to facilitate alignment of the fastener 200 with an opening of the locking hole 402. Locking hole 402 may be configured to receive a fixed or variable angle fastener 200. Locking hole 402 may be generally conical in shape such that it is wider near the top surface of plate 400 and narrower toward a bottom surface plate 400. The tapered portion and/or the textured portion 404 may be conical in shape.

In operation, shaft portion 204 may be threaded such that the fastener 200 may be threaded into the bone. The head portion 202 of the locking fastener 200 may include the textured area 206 around its outer surface that is sized and configured to engage with locking hole 402 of plate 400. Textured area 206 may include threads, ridges, bumps, dimples, serrations, or other types of textured areas. As shown, texture area 206 preferably includes a threaded portion extending substantially from the top of the head portion 202 to the bottom of the head portion 202 proximate to the shaft portion 204. The textured portion 404 of locking holes 402 may deform as head 202 interferes with the textured portion 404 of the hole 402, thereby providing a positive lock between the fastener 200 and the plate 400. Thus, as shown in FIG. 6, when locking fastener 200 is engaged with plate 400, textured area 206 is screwed into textured area 404 thereby deforming textured area 404 to lock fastener 200 into plate 400.

In an example, locking hole 402 may be configured to have a substantially smooth surface rather than having textured surface 404. In this embodiment, when locking fastener 200 is inserted into plate 400, textured area 206 digs into the substantially smooth inner surface of the locking hole 402 thereby locking fastener 200 into plate 400.

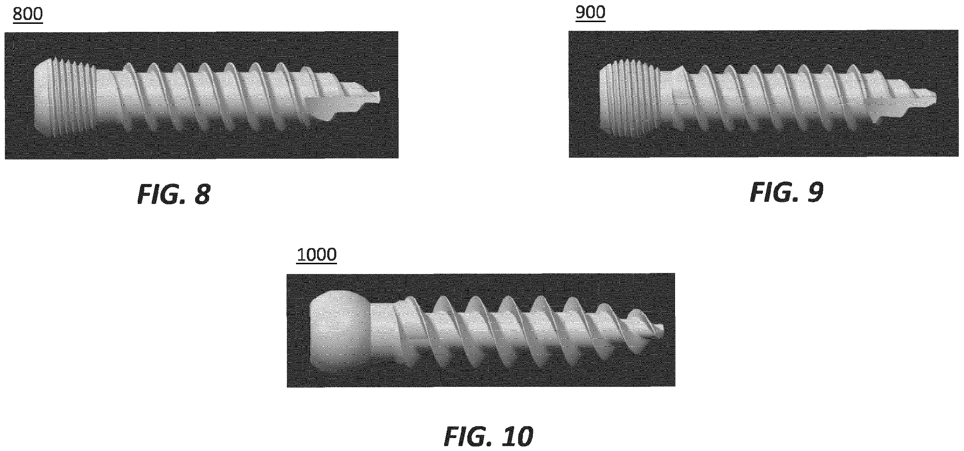

FIGS. 8-10 depict embodiments of bone screws 800, 900, and 1000 that may be used according to the principles of the present disclosure. Bone screws 800, 900, and 1000 may be a fixed or variable angle 5.5 mm outside diameter (O.D.) screw. The screws may be spherically or conically tapered dual lead threads intended to create a rigid connection with a smooth screw hole or screw hole modified with helical sweeps, diamond knurls, or similar relief cuts. The tapering dual-lead threads may be configured to lock into a plate or integrated plate-spacer to provide a rigid stable construct in weak or osteoporotic bone. Additionally, the screw minor diameter and pitch may be modified to improve purchase in weak bone. The screw material may be TAV or cobalt-chromium (CoCr). A blocking screw may also be used to promote screw retention and further minimize the chance of back-out.

FIG. 8 depicts a fastener 800 that is a spherical head with a conical taper of the dual leads. FIG. 9 depicts a fastener 900 that is a spherical head with spherical thread taper variant. FIG. 10 depicts a non-locking, non-self-drilling variant with smaller minor diameter than that of fasteners 800 and 900.

The locking screw feature described above combined with large cancellous threads of the screw may allow for a rigid connection of the screw to the implant in cases where weakened bone prevents lagging of bone onto the implant surface. The screw-implant construct provides greater stability in patients with poor bone quality.

The cutting/wedging behavior of the conical threads allows for the use of a locking-type screw in the same socket or screw hole geometry as a non-locking lagging screw that uses a blocking screw feature for retention. This offers greater versatility the surgeon in the types of screws they can used for fixation depending on patient anatomy and bone quality while not altering existing lag screws.

## Claims

1. A bone fixation system comprising:
- a bone plate (400) having an upper surface and a lower surface configured to be in contact with a bone, the bone plate (400) having locking hole (402) extending from the upper surface to the lower surface, the locking hole (402) including a textured portion (404), wherein the textured portion comprises a texture that is a non-threaded surface; and
- a locking fastener (200, 300, 800, 900, 1000), having a head portion (202, 302) and a shaft portion (204), configured to be received by the locking hole (402) and configured to be inserted into the bone, **characterised in that**
- the head portion (202, 302) is threaded and contains a textured area (206, 306) that is tapered and with dual lead threads and configured to engage the textured portion (404) of the locking hole (402), wherein an outside diameter of the locking fastener (200, 300, 800, 900, 1000) is 5.5 mm.

2. The bone fixation system of claim 1, wherein the textured portion (404) of the locking hole (402) is different from the textured area (206) of the locking fastener (200), so that when the locking fastener (200) is inserted into the locking hole (402), the locking fastener (200) deforms the textured portion (404).

3. The bone fixation system of claim 1, wherein the textured portion (404) take up about half or less of the surface area of the locking hole (402), the locking hole (402) comprising an upper portion that is tapered and without texturing to facilitate alignment of the fastener (200) with an opening of the locking hole (402).

4. The bone fixation system of claim 1, wherein the opening is generally conical in shape such that it is wider near the upper surface of the plate and narrower toward the lower surface of the plate.

5. The bone fixation system of claim 1, wherein the textured portion (404) comprises a knurled surface arranged about a central axis of the locking hole (402).

6. The bone fixation system of claim 1, wherein the textured portion (404) comprises diamond knurls arranged about a central axis of the locking hole (402).

7. The bone fixation system of claim 1, wherein the textured portion (404) comprises a knurled surface including a pattern of straight, angled, or crossed lines cut into the plate (400).

8. The bone fixation system of claim 1, wherein the threaded head portion (202, 206) is made of material that is harder than the textured area (404) of the locking hole (402).

9. The bone fixation system of claim 1, wherein the head portion (202) is conical.

10. The bone fixation system of claim 1, wherein the head portion (202) is spherical.

11. The bone fixation system of claim 1, further comprising a blocking screw configured to cover a portion of the locking fastener (200, 300, 800, 900, 1000) in a locked position to prevent the fastener from backing out of the plate (400).

## Patentansprüche

1. Knochenfixationssystem, umfassend:
- eine Knochenplatte (400) mit einer oberen Fläche und einer unteren Fläche, die eingerichtet sind, um mit einem Knochen in Kontakt zu stehen, wobei die Knochenplatte (400) ein Verriegelungsloch (402) aufweist, das sich von der oberen Fläche zur unteren Fläche erstreckt, wobei das Verriegelungsloch (402) einen texturierten Abschnitt (404) umfasst, wobei der texturierte Abschnitt eine Textur aufweist, die eine gewindelose Fläche ist; und
- ein Verriegelungsbefestigungselement (200, 300, 800, 900, 1000) mit einem Kopfabschnitt (202, 302) und einem Schaftabschnitt (204), das eingerichtet ist, um vom Verriegelungsloch (402) aufgenommen zu werden, und eingerichtet ist, um in den Knochen eingesetzt zu werden, **dadurch gekennzeichnet, dass**
- der Kopfabschnitt (202, 302) mit Gewinde versehen ist und einen texturierten Bereich (206, 306) aufweist, der sich verjüngend ist und zweigängige Gewinde aufweist, und eingerichtet ist, um mit dem texturierten Abschnitt (404) des Verriegelungslochs (402) in Eingriff zu kommen, wobei ein Außendurchmesser des Verriegelungsbefestigungselements (200, 300, 800, 900, 1000) 5,5 mm beträgt.

2. Knochenfixationssystem nach Anspruch 1, wobei der texturierte Abschnitt (404) des Verriegelungslochs (402) sich vom texturierten Bereich (206) des Verriegelungsbefestigungselements (200) unterscheidet, so dass, wenn das Verriegelungsbefestigungselement (200) in das Verriegelungsloch (402) eingesetzt wird, das Verriegelungsbefestigungselement (200) den texturierten Abschnitt (404) verformt.

3. Knochenfixationssystem nach Anspruch 1, wobei der texturierte Abschnitt (404) etwa die Hälfte oder weniger des Flächenbereichs des Verriegelungslochs (402) einnimmt, wobei das Verriegelungsloch (402) einen oberen Abschnitt umfasst, der sich verjüngend und ohne Texturierung ist, um eine Ausrichtung des Befestigungselements (200) mit einer Öffnung des Verriegelungslochs (402) zu ermöglichen.

4. Knochenfixationssystem nach Anspruch 1, wobei die Öffnung im Wesentlichen konisch geformt ist, so dass sie nahe der oberen Fläche der Platte breiter und zur unteren Fläche der Platte hin schmaler ist.

5. Knochenfixationssystem nach Anspruch 1, wobei der texturierte Abschnitt (404) eine gerändelte Fläche aufweist, die um eine Mittelachse des Verriegelungslochs (402) herum angeordnet ist.

6. Knochenfixationssystem nach Anspruch 1, wobei der texturierte Abschnitt (404) Diamant-Rändelungen aufweist, die um eine Mittelachse des Verriegelungslochs (402) herum angeordnet sind.

7. Knochenfixationssystem nach Anspruch 1, wobei der strukturierte Abschnitt (404) eine gerändelte Fläche aufweist, die ein Muster aus geraden, gewinkelten oder gekreuzten Linien umfasst, die in die Platte (400) geschnitten sind.

8. Knochenfixationssystem nach Anspruch 1, wobei der mit Gewinde versehene Kopfabschnitt (202, 206) aus einem Material hergestellt ist, das härter als der strukturierte Bereich (404) des Verriegelungslochs (402) ist.

9. Knochenfixationssystem nach Anspruch 1, wobei der Kopfabschnitt (202) konisch ist.

10. Knochenfixationssystem nach Anspruch 1, wobei der Kopfabschnitt (202) kugelförmig ist.

11. Knochenfixationssystem nach Anspruch 1, das ferner eine Blockierschraube aufweist, die eingerichtet ist, um einen Teil des Verriegelungsbefestigungselements (200, 300, 800, 900, 1000) in einer verriegelten Position abzudecken, um zu verhindern, dass sich das Befestigungselement aus der Platte (400) herausbewegt.

## Revendications

1. Système de fixation osseuse comprenant :
- une plaque osseuse (400) ayant une surface supérieure et une surface inférieure configurées pour venir au contact d'un os, la plaque osseuse (400) ayant un trou de verrouillage (402) s'étendant à partir de la surface supérieure jusqu'à la surface inférieure, le trou de verrouillage (402) incluant une partie texturée (404), dans lequel la partie texturée comprend une texture qui est une surface non filetée ; et
- une attache de verrouillage (200, 300, 800, 900, 1000), ayant une partie tête (202, 302) et une partie tige (204), configurée pour être reçue par le trou de verrouillage (402) et configurée pour être insérée dans l'os, **caractérisé en ce que**
- la partie tête (202, 302) est filetée et contient une zone texturée (206, 306) qui est fuselée et avec des filetages à double filet et configurée pour se mettre en prise avec la partie texturée (404) du trou de verrouillage (402), dans lequel un diamètre extérieur de l'attache de verrouillage (200, 300, 800, 900, 1000) est de 5,5 mm.

2. Système de fixation osseuse selon la revendication 1, dans lequel la partie texturée (404) du trou de verrouillage (402) est différente de la zone texturée (206) de l'attache de verrouillage (200), de telle sorte que lorsque l'attache de verrouillage (200) est insérée dans le trou de verrouillage (402), l'attache de verrouillage (200) déforme la partie texturée (404).

3. Système de fixation osseuse selon la revendication 1, dans lequel partie texturée (404) couvre environ la moitié ou moins de la surface du trou de verrouillage (402), le trou de verrouillage (402) comprenant une partie supérieure qui est fuselée et sans texturage pour faciliter l'alignement de l'attache (200) avec une ouverture du trou de verrouillage (402).

4. Système de fixation osseuse selon la revendication 1, dans lequel l'ouverture est généralement de forme conique de telle sorte qu'elle est plus large à proximité de la surface supérieure de la plaque et plus étroite en direction de la surface inférieure de la plaque.

5. Système de fixation osseuse selon la revendication 1, dans lequel la partie texturée (404) comprend une surface moletée agencée autour d'un axe central du trou de verrouillage (402).

6. Système de fixation osseuse selon la revendication 1, dans lequel la partie texturée (404) comprend des moletages en losange agencés autour d'un axe central du trou de verrouillage (402).

7. Système de fixation osseuse selon la revendication 1, dans lequel la partie texturée (404) comprend une surface moletée incluant un profil de lignes droites, angulaires ou croisées coupées dans la plaque (400).

8. Système de fixation osseuse selon la revendication 1, dans lequel la partie tête filetée (202, 206) est constituée d'un matériau qui est plus dur que la zone texturée (404) du trou de verrouillage (402).

9. Système de fixation osseuse selon la revendication 1, dans lequel la partie tête (202) est conique.

10. Système de fixation osseuse selon la revendication 1, dans lequel la partie tête (202) est sphérique.

11. Système de fixation osseuse selon la revendication 1, comprenant en outre une vis de blocage configurée pour recouvrir une partie de l'attache de verrouillage (200, 300, 800, 900, 1000) dans une position verrouillée pour empêcher que l'attache ne ressorte de la plaque (400).
